Europäisches Patentamt

European Patent Office

Office européen des brevets

(11) Publication number: **0 447 748 A1**

# EUROPEAN PATENT APPLICATION

(21) Application number: 91100399.4

(22) Date of filing: **15.01.91**

(51) Int. Cl.⁵: **A61N 1/30, A61N 1/44**

(30) Priority: **17.01.90 IT 4001090**

(43) Date of publication of application:
**25.09.91 Bulletin 91/39**

(84) Designated Contracting States:
**AT BE CH DE DK ES FR GB GR IT LI LU NL SE**

(71) Applicant: **Rossi, Cino**
**Via Settala 32**
**I-00123 Roma(IT)**

Applicant: **Eruzzi, Silvio**
**Via A. Mori 23**
**I-46100 Mantova(IT)**

(72) Inventor: **Rossi, Cino**
**Via Settala 32**
**I-00123 Roma(IT)**
Inventor: **Eruzzi, Silvio**
**Via A. Mori 23**
**I-46100 Mantova(IT)**

(74) Representative: **Modiano, Guido et al**
**MODIANO, JOSIF, PISANTY & STAUB**
**Modiano & Associati Via Meravigli, 16**
**I-20123 Milano(IT)**

(54) **Device for carrying out a method for the extracorporeal iontophoretic treatment of body parts having a substantially elongated shape.**

(57) The invention consists of a device (1) for carrying out a method for the iontophoretic treatment of external body parts which have a substantially elongated shape and is essentially constituted by a substantially cylindrical container (3) with an open top, in which an ionized medicated solution (4) is introduced; the base (3a) of the container (3) is made of a material which conducts low-voltage current and has elements (5) for connection to a related generator (G) and to a related grounding electrode (D); the container (3) is furthermore internally provided with safety devices (6) which can be interposed between an immersed body part (2) and the base (3a).

FIG.1

EP 0 447 748 A1

# METHOD FOR THE EXTRACORPOREAL IONTOPHORETIC TREATMENT OF BODY PARTS HAVING A SUBSTANTIALLY ELONGATED SHAPE AND DEVICE FOR CARRYING OUT THE METHOD

The present invention relates to a method for the extracorporeal iontophoretic treatment of body parts having a substantially elongated shape and to a device for carrying out the method.

Topical iontophoresis is currently performed by using devices manufactured with materials which imbibe and subsequently distribute medicated solutions; said materials, due to their intrinsic nature, do not allow capillary adhesion to the portion of skin to be treated.

This is the case, for example, of sponges and hides of various types, which when rested on the skin indeed have a contact therewith which is microscopically unstable due to the considerable percentage of empty spaces which exists between their fibers and which opposes complete and perfect adhesion between the surfaces involved.

This leads to a significant reduction in the effectiveness of the iontophoretic treatment, since the area of mutual contact between the surfaces is relatively limited and therefore the amount of medicated solution which is applied and subsequently activated with a low-voltage current on the diseased part is low.

Complete healing thus entails substantially long times, due to said limited effectiveness of the treatment, and this consequently affects the medical care expenses which medical organizations must face in order to assist patients requiring this therapy.

The technical aim of the present invention is to provide a method for the extracorporeal iontophoretic treatment of body parts having a substantially elongated shape and a device for carrying out the method which can considerably increase the surface contact between the medicated solution and the skin of the diseased part, all this translating into healing times which are considerably shorter than current ones and consequently into a significant saving in the costs which medical care must bear for each treated patient.

This aim and other objects are achieved by a method for the extracorporeal iontophoretic treatment of body parts having a substantially elongated shape, characterized in that it consists in immersing said part in an ionized medicated solution, in exciting the ions with a current having a voltage which is gradually adjustable and is in any case low, for times which can be adjusted as a function of the depth which said ions must reach in the tissues, said current originating from at least one external generator aided by a surface grounding electrode placed in contact with the skin, said excitation causing a concentrated and orientated flow of ions which penetrates the diseased and/or lacerated portion, which has a lower resistance to mass transfer with respect to the healthy portions.

Advantageously, the device for carrying out the above method is characterized in that it comprises a container for an ionized medicated solution, with a closeable top and with a substantially cylindrical shape; the base of said container is made of a material which conducts current at low voltage, has elements for connecting to a related generator and to a related grounding electrode and is internally provided with safety devices which can be interposed between an immersed body and said base.

Further characteristics and advantages of the invention will become apparent from the description of a preferred but not exclusive embodiment of a method for the extracorporeal iontophoretic treatment of body parts having a substantially elongated shape and of a device for carrying out said method, the practical execution whereof is represented in a preferential form in the accompanying drawing, wherein:

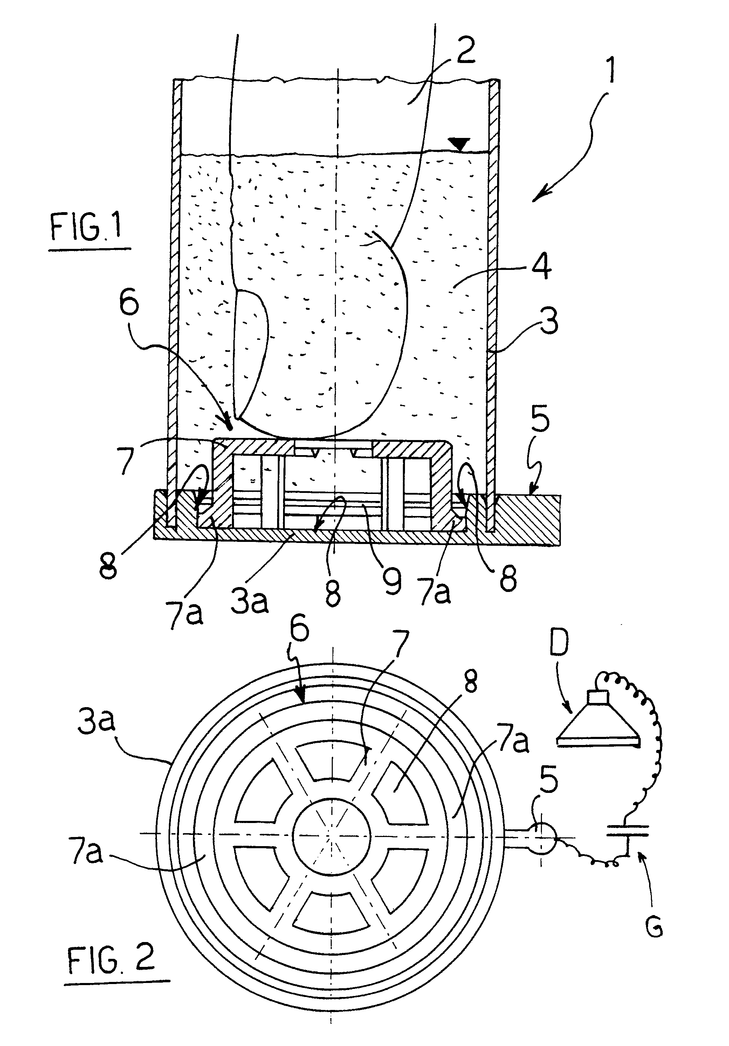

figure 1 is a schematic sectional view of a device for carrying out a method for the extracorporeal iontophoretic treatment of parts having a substantially elongated shape;

figure 2 is a top view of the device illustrated in figure 1.

With particular reference to the above figures, the reference numeral 1 generally indicates a device for carrying out a method for the extracorporeal iontophoretic treatment of body parts having a substantially elongated shape, in the specific case a finger 2.

Said device is essentially constituted by a body shaped like a container 3, with a closeable top and with a substantially cylindrical shape, for medicated solutions 4 which contain ions which can be activated with an electric excitation.

The base 3a of the container is made of conducting material and has elements 5 for connection to an external generator, schematically indicated by G, as well as to a grounding electrode D which can be applied on the surface of the patient's skin.

Said base 3a is provided, on the internal face of the container which acts as bottom of the container itself, with safety devices 6 which can be interposed between the immersed part (the finger 2) and said base 3a; said devices consist of a barrier which is shaped like a sort of latticed dome 7 which has, at its lower portion and along its entire external perimeter, a ridge 7a which can be inserted in a snug-fit manner in a related and complementarily ridged seat 8 defined in said base 3a.

The method according to the invention and the use of the device for its execution are as follows:
a preset volume of medicated solution is introduced into the container 3; the diseased part to be treated, which is constituted by a finger 2 in the illustrated example, is then immersed in said solution.

The grounding electrode D is furthermore arranged proximate to the region to be treated and is secured to the skin of the patient.

By activating the generator G, the ions present in the solution 4 are excited and most of them produce a substantially continuous and orientated flow which, due to the lower resistance to mass transfer which diseased or, even more so, lacerated tissues have with respect to healthy ones, penetrate them thoroughly, conveying the medication where it is strictly necessary.

Furthermore, the depth which said ions can reach in the tissues is adjusted either by acting on the intensity of the current supplied by the generator G or by extending or reducing the emission time of said current.

The immersion of the part to be treated makes certain that the mutual exchange surfaces between said part and the dissolved medication have no gaps and are therefore the largest possible.

Furthermore, the latticed dome barrier 7, made of thermoelectrically insulating material, is provided so as to prevent direct contact between the immersed part and the base 3a of the container 3 and therefore avoid any accidental burns; said barrier is arranged with a snug fit in the related seat 8 and is quite firm therein by virtue of the retention ridges 7a with which the lower portion of the dome is provided and by virtue of the complementarily shaped ridges 9 defined on the walls of said seat 8.

In practice it has been observed that the invention thus described achieves the intended aim and objects.

The invention thus conceived is susceptible to numerous modifications and variations, all of which are within the scope of the inventive concept.

All the details may furthermore be replaced with other technically equivalent elements.

In practice, the materials employed, as well as the dimensions, may be any according to the requirements without thereby abandoning the scope of the protection of the following claims.

Where technical features mentioned in any claim are followed by reference signs, those reference signs have been included for the sole purpose of increasing the intelligibility of the claims and accordingly such reference signs do not have any limiting effect on the scope of each element identified by way of example by such reference signs.

## Claims

1. Device for carrying out a method for the iontophoretic and extracorporeal treatment of body parts (2) having a substantially elongated shape, said method consisting in immersing said part (2) in an ionized medicated solution (4), in exciting the ions with a current having a voltage which is gradually adjustable and is low in any case, for times which can be adjusted as a function of the depth which said ions must reach in the tissues, said current originating from at least one external generator (G) aided by a surface grounding electrode (D) arranged in contact with the skin, said excitation producing a concentrated and orientated flow of ions which penetrates the diseased and/or lacerated portion, which has a lower resistance to mass transfer than healthy portions, said device being characterized in that it comprises a container (3), having a closeable top and a substantially cylindrical shape, for said ionized medicated solution (4), the base (3a) of said container being made of a material which conducts a low-voltage current, having elements (5) for connection to said related generator (G) and to said related grounding electrode (D), and being internally provided with safety devices (6) which can be interposed between said immersed body part (2) and said base (3a).

2. Device according to claim 1, characterized in that said connecting elements (5) comprise at least one connection terminal for the end of a power supply cable which is connected to said generator (G).

3. Device according to claim 1, characterized in that said safety devices (6) comprise a barrier which is shaped like a sort of latticed dome (7) which is provided, at its lower end, with an external ridge (7a) which can be inserted in a snug-fit manner in a related complementarily ridged seat (8) defined in said base (3a), said dome (7) being made of an electrically and thermally insulating material.

FIG.1

FIG. 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | <u>DE - A1 - 2 547 060</u> (HANIKA) * Page 2, lines 3-14; fig. 1 * | 1,2 | A 61 N 1/30 A 61 N 1/44 |
| A | <u>DE - A1 - 3 637 887</u> (SCHILLINGER) * Abstract; claims 1,18,26 * | 1,2 | |
| A | <u>FR - A1 - 2 403 085</u> (LE BOULANGER) * Page 2, lines 24-32; page 5, lines 3-30; fig. 5-9 * | 1,2 | |
| A | <u>FR - E - 46 813</u> (COMPAGNIE ELECTRO) * Fig. 1 * | 1-3 | |
| A | <u>US - A - 4 261 371</u> (READING) * Abstract; fig. 1 * | 1,2 | |

TECHNICAL FIELDS SEARCHED (Int. Cl.5)

A 61 N

The present search report has been drawn up for all claims

| Place of search | Date of completion of the search | Examiner |
|---|---|---|
| VIENNA | 25-04-1991 | NEGWER |

EPO FORM 1503 03.82 (P0401)